# EUROPÄISCHE PATENTANMELDUNG

(11) **EP 2 016 901 A1**
(43) Veröffentlichungstag der Anmeldung: **21.01.2009**
(21) Anmeldenummer: 08009791.8
(22) Anmeldetag: 29.05.2008
(51) Int. Cl.: A61B 6/00, H05G 1/06

(54) **Röntgenstrahlenquelle für eine mobile Röntgendiagnostikeinrichtung mit einem C-Bogen**

(30) Priorität: 08.06.2007 DE 102007026677
(71) Anmelder: Ziehm Imaging GmbH, 90451 Nürnberg (DE)
(72) Erfinder: Dehler, Juergen, 91301 Forchheim (DE); Guenther, Michael, 90451 Nürnberg (DE); Hoerndler, Klaus, 90482 Nürnberg (DE)

(57) **Zusammenfassung**

Die Erfindung betrifft eine Röntgenstrahlenquelle für eine mobile Röntgendiagnostikeinrichtung mit einem C-Bogen (60), wobei ein an das Hohlprofil (63) des C-Bogens angepaßter Generatorkessel (82) einen ersten Teilbereich mit einem Hochspannungsgenerator (83) und einen zweiten Teilbereich mit einer Röntgenröhre (81) aufweist und daß wenigstens der erste oder der zweite Teilbereich wenigstens teilweise im Hohlprofil (63) des C-Bogens (60) integriert ist.

## Beschreibung

Die Erfindung betrifft eine Röntgenstrahlenquelle für eine mobile Röntgendiagnostikeinrichtung mit einem C-Bogen

Aus der deutschen Patentschrift DE 44 23 359 B4 der Anmelderin ist eine mobile Röntgendiagnostikeinrichtung mit einem mehrfach verstellbaren C-Bogen mit an den Enden des C-Bogens einander gegenüber angeordneten Röntgenstrahlenquelle und einem Röntgenstrahlenempfänger bekannt, wobei alle Verstellachsen mit Inkrementalgebern ausgestattet sind. Eine solche Röntgendiagnostikeinrichtung erlaubt die Aufnahme einer Reihe von 2D-Röntgenprojektionsaufnahmen mit unterschiedlicher, bekannter Projektionsgeometrie, aus denen ein 3D-Röntgenmodell rekonstruiert werden kann. Nachteilig ist dabei, daß der Verfahrbereich des C-Bogens längs seines Umfanges (Orbitalbewegung) jeweils etwa nur 130 ° beträgt. Würde durch konstruktive Maßnahmen bei sonst gleichen Abmessungen des C-Bogens der Winkelbereich der Orbitalbewegung auf gewünschte mindestens 180° vergrößert werden, so würde die Öffnungsweite zwischen Röntgenstrahlenquelle und Röntgenstrahlenempfänger verkleinert werden, was die Positionierbarkeit der mobilen Röntgendiagnostikeinrichtung stark einschränken würde. Die Möglichkeit, gleichzeitig auch den Durchmesser des C-Bogens zu vergrößern, ist bei mobilen Geräten wegen der Gewichtszunahme und der dadurch bedingten wachsenden mechanischen Instabilität nicht das Mittel der Wahl.

Aus der US-Patentschrift US 6 880 691 B2 und aus der französischen Offenlegungsschrift FR 2 671 507 A sind Röntgendiagnostikeinrichtungen mit C-Bögen bekannt, bei denen die Röntgenstrahlenquelle und der Röntgenstrahlenempfänger innerhalb des jeweiligen C-Bogens angeordnet sind.

Aus der US-Patentschrift US 6 364 526 B2 und aus der französischen Offenlegungsschrift FR 2 342 704 A sind Röntgendiagnostikeinrichtungen bekannt, bei denen der Röntgenstrahler und die Röntgenstrahlenquelle seitlich am C-Bogen angeordnet sind und soweit vom Mittelpunkt des C-Bogens nach außen versetzt werden können, daß die Öffnungsweite durch diese Komponenten nicht beeinträchtigt wird. Diese asymmetrische Anordnung weist jedoch den Nachteil auf, daß die seitliche Ausdehnung des C-Bogens mit der Röntgenstrahlenquelle vergrößert wird und die Positionierbarkeit, insbesondere bei Unter-Tisch-Anwendungen erschwert wird.

Aus der Patentliteratur sind mobile Röntgenstrahlenquellen bekannt, bei denen eine Röntgenröhre, vorzugsweise in einem Isolierölbehälter, durch ein Hochspannungskabel mit einem Hochspannungsgenerator verbunden ist. Als Beispiel sei die GB 351 534 A angeführt. Die verwendeten Hochspannungskabel sind insbesondere für Spannungen im Bereich von über 80 kV wenig flexibel und sind bei den beengten Platzverhältnissen im Inneren einer mobilen Röntgendiagnostikeinrichtung schlecht unterzubringen.

Aus der DE 198 24 008 C2 ist ein kompakter Röntgenstrahler für eine mobile Röntgendiagnostikeinrichtung bekannt, der in einem Öl gefüllten Kessel eine Röntgenröhre und einen Hochspannungsgenerator aufweist und der an einem Ende des C-Bogens und außerhalb des Bereiches der Führungsschienen in Umfangsrichtung angeordnet ist.

Aufgabe der Erfindung ist es, eine kompakte Röntgenstrahlenquelle mit einem Generatorkessel für eine mobile Röntgendiagnostikeinrichtung mit einem C-Bogen mit Hohlprofil zu schaffen, bei der der Abstand der Röntgenstrahlenquelle vom Mittelpunkt des C-Bogens gegenüber den bekannten Anordnungen bei gleichem C-Bogen-Durchmesser verringert ist.

Die Aufgabe der Erfindung wird durch eine Röntgenstrahlenquelle gelöst, die einen an das Hohlprofil des C-Bogens angepaßten Generatorkessel mit einem ersten Teilbereich, der eine Röntgenröhre (81) enthält und einem zweiten Teilbereich, der einen Hochspannungsgenerator enthält, aufweist und der mit wenigstens dem ersten oder dem zweiten Teilbereich wenigstens teilweise im Hohlprofil des C-Bogens integriert ist.

Die erfindungsgemäße Röntgenstrahlenquelle wird an Hand der Abbildungen näher erläutert.

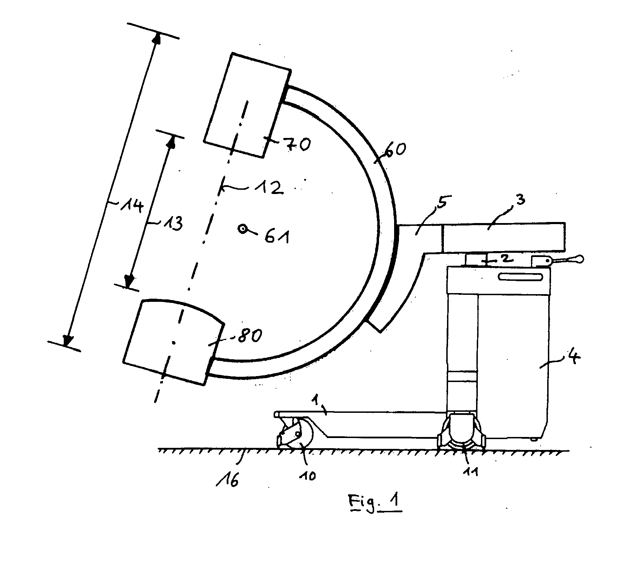

In Fig. 1 ist ein C-Bogen nach dem Stand der Technik schematisch dargestellt. An einem annähernd kreisbogenförmigen C-Bogen (60) sind gegenüberliegend am einen Ende eine Röntgenstrahlenquelle (80) und am anderen Ende ein Röntgenstrahlenempfänger (70) angeordnet. Der C-Bogen (60) ist längs seines Umfangs in einer Halterung 5 verschieblich gelagert, welche selbst an einer vertikalen Säule 2 höhenverstellbar gelagert ist. Die Führung der Säule 2 ist starr mit einem Fahrgestell 1 verbunden, der auch den Geräteschrank 4 trägt. Das Fahrgestell 1 weist Rollen 10, 11 auf, die auf dem Fußboden 16 verfahrbar sind. Die in Fig. 1 dargestellte Röntgendiagnostikeinrichtung weist eine Öffnungsweite (13) und eine Außenabmessung (14) auf. Für eine gute Positionierbarkeit des Röntgendiagnostikeinrichtung ist eine möglichst große Öffnungsweite (13) und eine möglichst kleine Außenabmessung (14) wünschenswert. Dies wird beispielsweise durch Verwendung eines bildgebenden Festkörperdetektors (Flat Panel Detektor, FPD) oder/und durch Veränderungen an der Röntgenstrahlenquelle (80) erreicht.

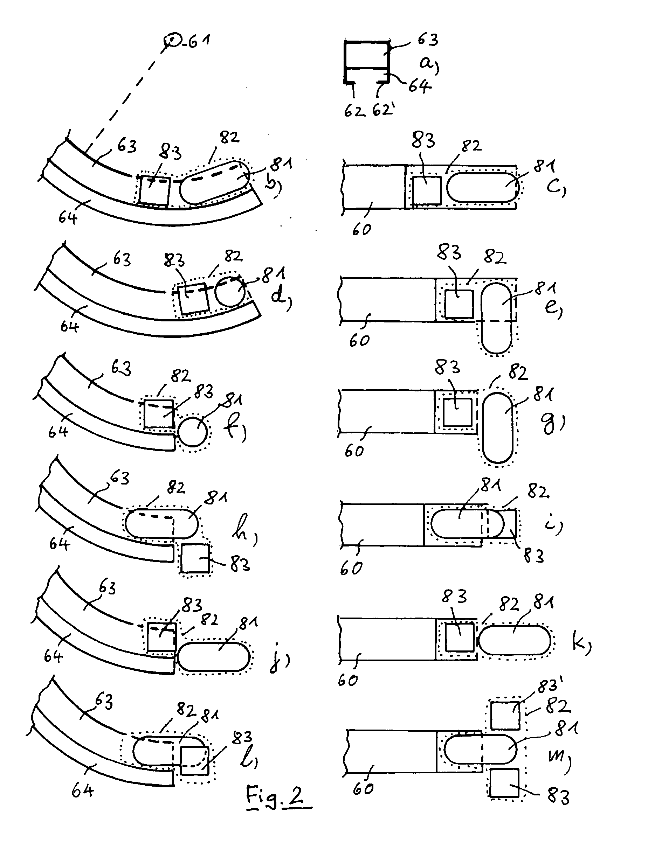

In Fig. 2a ist ein Profil eines C-Bogens im Schnitt dargestellt, wie es beispielsweise aus der deutschen Offenlegung DE 196 30 888 A1 bekannt ist. Es weist ein Hohlprofil (63) auf, an das sich ein im wesentlichen u-förmiges Profil (64) mit den Führungsschienen (62, 62') anschließt, an welchen der C-Bogen (60) an der Halterung (5) verschieblich gelagert ist.

In den Abbildungen Fig. 2b bis 2m sind im Schnitt (Fig. 2b, d, f, h, j, l) und in der Aufsicht (Fig. 2c, e, g, i, k, m) verschiedene Ausprägungen der erfindungsgemäßen Röntgenstrahlenquelle und ihre Anordnung am C-Bogen (60) schematisch dargestellt. Dabei ist jeweils rechts von einer Schnittdarstellung die korrespondierende Ansicht dargestellt.

Im Beispiel der Fig. 2 ist bei allen gezeigten Ausprägungen der Erfindung eine Röntgenröhre (81) und ein Hochspannungsgenerator (83) in einem, diese gemeinsam umschließenden, mit Isolieröl gefüllten Generatorkessel (82) angeordnet, dessen Wandung schematisch als punktierte Linie dargestellt ist. Die elektrischen Verbindungen zwischen dem Hochspannungsgenerator (83) und der Röntgenröhre (81) sind nicht dargestellt, ebensowenig elektrische, optische oder hydraulische Verbindungsleitungen zwischen dem Generatorkessel (82) und dem Geräteschrank (4) der Röntgendiagnostikeinrichtung. Innerhalb des Generatorkessels sind nicht dargestellte Mittel zur Unterstützung der Konvektion und zum Abtransport der beim Betrieb erzeugten Verlustwärme vorgesehen. Der Generatorkessel weist zwei miteinander verbundene Teilbereiche auf, von denen der erste Teilbereich die Röntgenröhre (81) und der zweite Teilbereich den Hochspannungsgenerator (83) in der gemeinsamen Isolierölfüllung des Generatorkessels (82) enthält. Wenigstens einer der beiden Teilbereiche des Generatorkessels (82) ist wenigstens teilweise innerhalb des Hohlprofils (63) des C-Bogens (60) angeordnet.

Im Rahmen der Erfindung ist es ferner vorgesehen, den Generatorkessel (82) aus zwei voneinander lösbaren Teilen aufzubauen, wobei ein erster Teil mit dem Hochspannungsgenerator (83) eine Isolierölfüllung und wenigstens zwei Hochspannungsdurchführungen aufweist, diejenige Kesselwand des ersten Teils hindurch führen, der an einen zweiten, die Röntgenröhre (81) aufweisenden Teil angrenzt, wobei der zweite Teil eine separate Isolierölfüllung aufweist. Es ist vorgesehen, daß der zweite Teil des Generatorkessel (82) kann ebenso wenigstens zwei Hochspannungsdurchführungen aufweist, die mit den Hochspannungsdurchführungen des ersten Teils durch eine Steckverbindung lösbar verbunden sind. Wenn im Servicefall die Röntgenröhre ausgetauscht werden müßte, würde der zweite Teil des Generatorkessels vom ersten gelöst werden, ohne daß die Abdichtung einer Isolierölfüllung verändert werden müßte.

Es ist alternativ vorgesehen, den ersten Teil des Generatorkessels (82) mit wenigstens zwei Hochspannungsdurchführungen auszustatten und den zweiten Teil mit der Röntgenröhre (81) auf der dem ersten Teil zugewandten Seite mit wenigstens einer Öffnung im Bereich der Hochspannungsdurchführungen zu versehen und diese wenigstens eine Öffnung in der Wand des zweiten Teils durch die Wand des ersten Teils, durch die Hochspannungsdurchführungen und durch eine zwischen dem ersten und dem zweiten Teil angeordnete umlaufende Dichtung zu verschließen. Im Servicefall würde die Röntgenröhre (81) an die Hochspannungsdurchführungen angeschlossen werden und anschließend der zweite Teil mit Isolieröl gefüllt werden verschlossen wird.

Es ist vorgesehen, wenigstens in der Isolierölfüllung des zweiten Teils des Generatorkessels (82) Mittel zur Kühlung der Isolierölfüllung, insbesondere Wärmetauscher anzuordnen. Sind in der Isolierölfüllung mehrere Wärmetauscher vorgesehen, so ist vorgesehen, diese außerhalb des Generatorkessels (82) miteinander zu verbinden um eine wie Parallel- und/oder Hintereinanderschaltung der Wärmetauscher zu ermöglichen.

Ist als Röntgenröhre (81) eine Drehanodenröhre vorgesehen, so besitzt diese während des Betriebs einen Drehimpuls, wodurch bei Änderung der Richtung des Drehimpulsvektors im Gravitationsfeld der Erde eine Kraft senkrecht zu der Rotationsachse der Drehanodenröhre wirkt. Insbesondere bei schnellen Orbitalbewegungen im Rahmen von Scans kann es dabei zu Veränderungen der Röntgenprojektionsgeometrie kommen, die im quasistischen Falle nicht auftreten. Deshalb sind für Röntgendiagnostikeinrichtungen, die für schnelle Scans ausgelegt sind, die Anordnungen der Röntgenröhre (81) gemäß der Fig. 2d bis 2g unter gewissen Umständen einer Orientierung der Drehachse der Drehanodenröhre senkrecht zu der Ebene des C-Bogens (60) vorzuziehen.

Wie in Fig. 2b bis 2e dargestellt, können der erste und der zweite Teilbereich des Generatorkessels (82) teilweise innerhalb des Hohlprofils (63) angeordnet sein. Dabei wird die dem Mittelpunkt (61) des C-Bogens (60) zugewandte Seite des Hohlprofils (63) im Bereich des Generatorkessels entfernt; bei Platzbedarf auch Teile der Seitenwangen. Würde durch das Aufbrechen des Hohlprofils (63) im Bereich des Generatorkessels (82) die Stabilität des C-Bogens (60) geschwächt werden, so sind Verstärkungen am C-Bogen in diesem Bereich vorgesehen.

Bei den schematischen Darstellungen der Fig. 2b bis 2m ist eine Verkleidung der Röntgenstrahlenquelle nicht dargestellt.

Der Generatorkessel (82) kann aus Blech geschweißt oder als Druckgußteil aus einer Leichtmetall-Legierung ausgeführt sein. Es ist auch vorgesehen, den Generatorkessel als Blasformteil mit Verbundwerkstoffen auszuführen.

In der Anmeldung zitierte Patentdokumente:
DE 44 23 359 B4
US 6 880 691 B2
FR 2 671 507 A
US 6 364 526 B2
FR 2 342 704 A
GB 351 534 A
DE 198 24 008 C2

### Verzeichnis der Abbildungen:

- Fig. 1:: Röntgendiagniostikeinrichtung (Stand der Technik)
- Fig. 2:: C-Bogen mit erfindungsgemäßer Röntgenstrahlenquelle

### Bezugszeichenliste:

- 1: Fahrgestell
- 2: Säule
- 3: Horizontalführung
- 4: Geräteschrank
- 5: Halterung
- 10: Rolle
- 11: Rolle
- 12: Zentralstrahl
- 13: Öffnungsweite
- 14: Außenmaß
- 16: Fußboden
- 60: C-Bogen
- 61: Mittelpunkt
- 62, 62': Führungsschienen
- 63: Hohlprofil
- 64: u-förmiges Profil
- 70: Röntgenstrahlenempfänger
- 80: Röntgenstrahlenquelle
- 81: Röntgenröhre
- 82: Generatorkessel
- 83, 83': Hochspannungsgenerator

## Patentansprüche

1. Röntgenstrahlenquelle mit einem Generatorkessel (82), mit einer in Isolieröl gelagerten Röntgenröhre (81) und einem in Isolieröl gelagerten Hochspannungsgenerator (83) für eine mobile Röntgendiagnostikeinrichtung mit einem C-Bogen (60), welcher aus einem Hohlprofil (63) und einem u-förmigen Profil (64) besteht,
**dadurch gekennzeichnet, daß** der Generatorkessel (82) eine an das Hohlprofil (63) des C-Bogens (60) angepaßte Form mit einem ersten Teilbereich, der den Hochspannungsgenerator (83) enthält und einem zweiten Teilbereich, der die Röntgenröhre (81) enthält, aufweist und daß wenigstens der erste oder der zweite Teilbereich wenigstens teilweise im Hohlprofil (63) des C-Bogens (60) integriert ist

2. Röntgenstrahlenquelle nach Anspruch 1,
**dadurch gekennzeichnet, daß** der Generatorkessel (82) eine einzige Isolierölfüllung, in der sowohl der Hochspannungsgenerator (83) als auch die Röntgenröhre (81) angeordnet sind.

3. Röntgenstrahlenquelle nach Anspruch 1,
**dadurch gekennzeichnet, daß** der Generatorkessel (82) zwei voneinander lösbare Teile aufweist, wobei der erste Teil den ersten Teilbereich und der zweite Teil den zweiten Teilbereich aufnimmt und wobei der erste Teil mit dem Hochspannungsgenerator (83) eine Isolierölfüllung und wenigstens zwei Hochspannungsdurchführungen durch die an den zweiten Teil angrenzende Kesselwand aufweist und wobei der zweite Teil eine separate Isolierölfüllung aufweist.

4. Röntgenstrahlenquelle nach Anspruch 3,
**dadurch gekennzeichnet, daß** der zweite Teil des Generatorkessel (82) wenigstens zwei Hochspannungsdurchführungen aufweist, die mit den Hochspannungsdurchführungen des zweiten Teils durch eine Steckverbindung lösbar verbunden sind.

5. Röntgenstrahlenquelle nach Anspruch 3,
**dadurch gekennzeichnet, daß** der zweite Teil des Generatorkessel (82) auf der dem ersten Teil zugewandten Seite wenigstens eine Öffnung im Bereich der Hochspannungsdurchführungen aufweist, und daß im montierten Zustand diese Öffnung in der Wand des zweiten Teils durch die Wand und die Hochspannungsdurchführungen des ersten Teils und durch eine zwischen dem ersten und dem zweiten Teil angeordnete umlaufende Dichtung verschlossen ist.

6. Röntgenstrahlenquelle nach einem der Ansprüche 1-5,
**dadurch gekennzeichnet, daß** wenigstens in der Isolierölfüllung des zweiten Teils des Generatorkessels (82) Mittel zur Kühlung der Isolierölfüllung angeordnet sind.
